# EUROPÄISCHE PATENTSCHRIFT

(11) **EP 0 597 076 B1**
(45) Veröffentlichungstag und Bekanntmachung des Hinweises auf die Patenterteilung: **02.12.1998**
(21) Anmeldenummer: 93912848.4
(22) Anmeldetag: 01.06.1993
(51) Int. Cl.: C12Q 1/68

(54) **SIMULTANE SEQUENZIERUNG VON NUKLEINSÄUREN**
SIMULTANEOUS SEQUENCING OF NUCLEIC ACIDS
SEQUENCAGE SIMULTANEE D'ACIDES NUCLEIQUES

(30) Priorität: 02.06.1992 DE 4218152
(43) Veröffentlichungstag der Anmeldung: 18.05.1994
(73) Patentinhaber: BOEHRINGER MANNHEIM GMBH, 68298 Mannheim (DE)
(72) Erfinder: SAGNER, Gregor, D-8122 Penzberg (DE); Kessler, Christoph, D-8021 Dorfen (DE); BLUM, Helmut, D-8000 München (DE); DOMDEY, Horst, D-8027 Neuried (DE)
(74) Vertreter: Huber, Bernhard, Dipl.-Chem.
(86) Internationale Anmeldenummer: EP9301376
(87) Internationale Veröffentlichungsnummer: WO9324654

(56) Entgegenhaltungen:
- EP-A- 0 093 948
- EP-A- 0 224 126
- EP-A- 0 303 459
- WO-A-92/22650
- DE-A- 4 011 991
- NUCLEIC ACIDS RESEARCH Bd. 19, Nr. 12, Juni 1991, ARLINGTON, VIRGINIA US Seiten 3301 - 3305 M. CHEE 'Enzymatic multiplex DNA sequencing'

## Beschreibung

Die vorliegende Erfindung betrifft ein neues Verfahren zur simultanen Sequenzierung von Nukleinsäuren sowie ein Verfahren zur Umwandlung einer bereits vorhandenen Genbank in eine modifizierte Genbank, an der eine simultane Sequenzierung von Nukleinsäuren durchführbar ist.

Die Sequenz von Nukleinsäuren wird im allgemeinen entweder durch chemische DNA-Sequenzierung (Maxam-Gilbert-Technik) oder durch das enzymatische Kettenabbruchverfahren (Sanger-Technik) bestimmt. Bei der Maxam-Gilbert-Technik erfolgt eine basenspezifischen Spaltung der zu sequenzierenden Nukleinsäure mit Hilfe von bestimmten Chemikalien. Bei der Sanger-Technik erfolgt eine enzymatische Polymerisationsreak-tion unter Verwendung der zu sequenzierenden Nukleinsäure als Matrize und einer DNA-Polymerase, z.B. des Klenow-Fragments der E.coli-DNA-Polymerase, der T4-DNA-Polymerase oder der T7-DNA-Polymerase. Als Substrat für das Enzym werden zusätzlich zu den normalen Desoxynukleosidtriphosphaten (dNTP) als Kettenabbruchmoleküle 2',3'-Didesoxynukleosid-5'-triphosphate (ddNTP) verwendet. Durch Einbau dieser Didesoxyverbindungen in einen frisch synthetisierten DNA-Strang wird ein Abbruch der Polymerisationsreaktion bewirkt.

Die Reihenfolge der einzelnen Arbeitsschritte ist bei beiden oben genannten Techniken im wesentlichen gleich: Zunächst wird eine zu sequenzierende, hochmolekulare DNA in mehrere kleinere Fragmente zerlegt, deren Sequenz dann bestimmt werden kann. Diese Zerlegung kann beispielsweise durch spezifische Spaltung mit Restriktionsenzymen oder auch durch unspezifische Spaltung mit DNaseI oder durch Ultraschallbehandlung der DNA erfolgen.

Die eigentliche Bestimmung der Sequenz erfolgt an den kleineren Fragmenten, wobei das Prinzip beider Sequenzierverfahren darin besteht, daß durch eine chemische oder enzymatische Reaktion eine Population von unterschiedlich langen Sequenzierungsprodukten erzeugt wird, deren Nukleotidsequenz sich aus der zu sequenzierenden DNA ableitet. Das eine Ende dieser Sequenzierungsprodukte ist für die ganze Population identisch und das andere Ende ist ein variables Ende mit jeweils einer der vier möglichen Basen der DNA. Durch Größenauftrennung, im allgemeinen durch Gelelektrophorese in sehr dünnen, hochauflösenden denaturierenden Polyacrylamidgelen, werden dann die unterschiedlich langen Sequenzierungsprodukte voneinander getrennt.

Die Ermittlung der Sequenz erfolgt im allgemeinen durch direkte Auswertung dieser Sequenzgele. In diesem Fall müssen die Sequenzierungsprodukte eine direkte Markierung, z.B. durch Einbau von Radioisotopen wie etwa ³²P oder ³⁵S oder biotinylierten oder fluoreszenzmarkierten Nukleotiden, aufweisen.

Der Nachteil einer direkten Markierung der Sequenzierungsprodukte ist, daß immer nur jeweils ein einziges DNA-Fragment bei jeder basenspezifischen Sequenzierungsreaktion bearbeitet werden kann. Dieser Nachteil kann durch eine simultane Sequenzierung von Nukleinsäuren nach der sogenannten Multiplex-Technik (siehe z.B. EP-A 0 303 459) beseitigt werden. Diese Technik erlaubt die gleichzeitige Bearbeitung von mehreren, im allgemeinen bis zu 50 unterschiedlichen DNA-Fragmenten bei jeder basenspezifischen Sequenzierungsreaktion. Dazu werden die zu sequenzierenden DNA-Fragmente in unterschiedliche Klonierungsvektoren eingebaut. Diese Vektoren unterscheiden sich jeweils dadurch, daß links und rechts von der Klonierungsstelle unterschiedliche, für den jeweils verwendeten Vektor spezifische Oligonukleotidsequenzen liegen. Alle Vektoren tragen außerhalb dieser Oligonukleotidsequenzen die gleiche Schnittstelle für ein Restriktionsenzym, mit dem die einklonierten DNA-Fragmente zusammen mit den für sie spezifischen Nachbarregionen wieder aus dem Vektor herausgeschnitten werden können. An diesem Fragmentgemisch wird eine Maxam-Gilbert-Sequenzreaktion durchgeführt. Nach gelelektrophoretischer Auftrennung der Sequenzierungsprodukte werden die Banden aus dem Sequenzgel auf eine Nylonmembran übertragen und dort immobilisiert. Durch Hybridisierung dieser Membran mit jeweils für einen einzigen Vektor spezifischen Hybridisierungssonden können nacheinander auf der gleichen Membran die Sequenzen mehrerer DNA-Fragmente sichtbar gemacht werden. Der Vorteil dieser Methode liegt darin, daß die vier basenspezifischen Sequenzierungsreaktionen und die Auftrennung der dadurch erhaltenen Sequenzierungsprodukte nur ein einziges Mal durchgeführt werden muß.

Ein Nachteil dieser Strategie besteht jedoch darin, daß zur Einklonierung der DNA-Fragmente eine Vielzahl von Vektoren mit jeweils unterschiedlichen spezifischen Oligonukleotidsequenzen erforderlich sind. Ein weiterer Nachteil des Multiplex-Verfahrens besteht darin, daß die Klonierung der DNA in die einzelnen Vektoren ausschließlich durch eine Blunt-End-Ligation möglich ist. Bei dieser Art der Klonierung ist nicht nur die Klonierungseffizienz vergleichsweise niedrig, sondern ein bestimmter Prozentsatz der Kolonien enthält aufgrund der niedrigen Ligationseffizienz reine Vektor-DNA. Dieser Anteil muß vor der Sequenzierung durch eine präparative Gelelektrophorese abgetrennt werden.

EP-A-0 224 126 betrifft ein Verfahren zur DNA-Sequenzierung unter Verwendung des Kettenabruchverfahrens, umfassend Replizieren einer Templatkette mit Nukleotidgemischen worin an die Kettenenden der zu sequenzierenden DNA-Fragmente ein Splinker gebunden wird, der einen komplementären Abschnitt von mindestens 4 Basen und eine selektiv spaltbare Verknüpfung aufweist. Bei diesen Verfahren wird die Splinker-verknüpfte doppelsträngige DNA denaturiert, um eine einzelsträngige DNA als Templatfragment zu erhalten, wobei der Splinker als Primer wirkt, welcher zur Sequenzierung der Nukleotidgemische enzymatisch verlängert wird.

Aufgrund dieser oben genannten Nachteile findet die Multiplex-Sequenzierungsstrategie nur eine relativ beschränkte Anwendung. Aufgabe der vorliegenden Erfindung war es somit, ein Verfahren zur simultanen Sequenzierung von Nukleinsäuren zu entwickeln, bei dem die oben genannten Nachteile, insbesondere die Verwendung einer Vielzahl von Basisvektoren und die geringe Klonierungseffizienz beseitigt sind.

Die erfindungsgemäße Aufgabe wird gelöst durch ein Verfahren zur simultanen Sequenzierung von Nukleinsäuren, welches gekennzeichnet ist durch
(a) Bereitstellen einer Vielzahl DNA-Fragmente, deren Sequenz bestimmt werden soll,
(b) Aufteilen der Gesamtheit der zu sequenzierenden DNA-Fragmente in mehrere Gruppen,
(c) Ligieren von doppelsträngigen DNA-Adaptoren an beide Enden der aufgeteilten DNA-Fragmente aus (b), wobei die Adaptoren einen doppelsträngigen Bereich mit einer Länge von ≥ 5 Nukleotiden besitzen, ein erstes zu den Enden der DNA-Fragmente kompatibles Ende und ein zweites, zur Klonierung in einen Vektor geeignetes Ende aufweisen, und wobei an jede Gruppe der aufgeteilten DNA-Fragmente Adaptoren mit unterschiedlicher Nukleotidsequenz ligiert werden,
(d) Klonieren der an beiden Enden mit Adaptoren versehenen DNA-Fragmente aus (c) in einen Basisvektor, der zu den zweiten Enden der Adaptoren passende Restriktionsstellen aufweist,
(e) Auswählen einer Anzahl von Klonen aus (d), wobei jedoch aus einer Gruppe nur maximal 1 Klon stammen darf, und Durchführen von Sequenzierungsreaktionen an den ausgewählten Klonen, und
(f) Auswerten der Ergebnisse der Sequenzierungsreaktionen unter Verwendung einer Anzahl von nachweisbaren Hybridisierungssonden, die jeweils nur für einen einzigen Klon aus (e) spezifisch sind.

Die Vorteile des erfindungsgemäßen Verfahrens gegenüber dem bekannten Multiplex-Verfahren bestehen insbesondere darin, daß im Gegensatz zu dem bekannten Multiplex-System nur ein einziger Basisvektor benötigt wird. Deshalb können die Adaptorsequenzen, welche im letzten Reaktionsschritt die Bindung der jeweils verwendeten Hybridisierungssonde an die immobilisierte Nukleinsäure bestimmen, bereits außerhalb des Vektors an die zu sequenzierenden DNA-Fragmente ligiert werden. Somit kann die Gesamtheit der zu sequenzierenden DNA-Fragmente in eine beliebige Anzahl von Gruppen aufgeteilt werden, da die Zahl dieser Gruppen nicht durch die Anzahl der vorhandenen Vektoren festgelegt wird. Die benötigten unterschiedlichen Adaptor-Oligonukleotide, die vorzugsweise 10 bis 40 Nukleoide lang sind, können auf einfache Weise mit beliebigen Sequenzen chemisch synthetisiert werden. Die Effizienz der Ligation der DNA-Fragmente mit den Adaptormolekülen kann durch einen Überschuß an eingesetzten Adaptormolekülen erheblich verbessert werden. Auch dies bewirkt höhere Ausbeuten an erhaltenen Kolonien pro eingesetzter DNA im Vergleich zum bekannten Multiplex-System.

Die gemäß Schritt (a) des erfindungsgemäßen Verfahrens bereitgestellten DNA-Fragmente können glatte oder gestufte Enden aufweisen. DNA-Fragmente mit glatten Enden sind z.B. durch Spaltung der DNA und gegebenenfalls anschließendes Glätten der Enden auf beliebige Weise erhältlich. Vorzugsweise werden diese DNA-Fragmente durch Zertrümmern von DNA mittels Ultraschall und enzymatischer Behandlung (z.B. mit T4-DNA-Polymerase oder Klenow-Polymerase) der Enden, Spaltung mit glattschneidenden Restriktionsenzymen, mechanische Scherung von DNA oder/und durch DNase-Behandlung erzeugt. DNA-Fragmente mit gestuften Enden werden vorzugsweise durch Spaltung von DNA mit entsprechenden Restriktionsenzymen oder/und anderen sequenzspezifisch schneidenden Enzymen erzeugt.

Schritt (b) des erfindungsgemäßen Verfahrens umfaßt das Aufteilen der Gesamtheit der zu sequenzierenden DNA-Fragmente in mehrere Gruppen. Die Anzahl dieser Gruppen kann an sich beliebig sein und hängt beispielsweise von der Größe der gesamten zu sequenzierenden DNA ab. Beispielsweise können die zu sequenzierenden DNA-Fragmente in 10 bis 1.000, vorzugsweise 50 bis 500 Gruppen oder Fraktionen aufgeteilt werden.

Diese einzelnen Gruppen von DNA-Fragmenten werden gemäß Schritt (c) des erfindungsgemäßen Verfahrens aus doppelsträngigen DNA-Adaptoren ligiert, die auf einer Seite ein erstes Ende aufweisen, das zu den DNA-Fragmenten kompatibel ist. Dieses Ende kann - wie zuvor ausgeführt - glatt oder gestuft sein. Auf ihrer anderen Seite weisen die Adaptoren ein zweites Ende auf, das zur Klonierung in einen Vektor geeignet ist. Dieses Ende kann glatt sein, es ist jedoch vorzugsweise gestuft, da in diesem Fall die Klonierung der zu sequenzierenden DNA-Fragmente in den Basisvektor nicht über eine Blunt-End-Ligation, sondern über eine Ligation von DNA-Fragmenten mit gestuften Enden erfolgt, so daß eine wesentlich bessere Klonierungseffizienz gefunden wird.

Der doppelsträngige Bereich eines Adaptors hat zweckmäßigerweise eine ausreichende Länge, um eine Ligation des Adaptors an das DNA-Fragment zu ermöglichen. Im allgemeinen muß der doppelsträngige Bereich daher ≥ 5 Nukleotide, vorzugsweise ≥ 6 Nukleotide lang sein.

An jede Gruppe der aufgeteilten DNA-Fragmente werden Adaptoren mit unterschiedlicher Nukleotidsequenz ligiert. Dabei kann man innerhalb einer Gruppe entweder einen einzigen Adaptor oder aber auch ein Gemisch aus zwei Adaptoren einsetzen, die sich in ihrer Nukleotidsequenz oder/und in ihrem zweiten (zur Klonierung in den Vektor vorgesehenen) Ende unterscheiden. In diesem Zusammenhang bedeutet der Begriff "unterschiedliche Nukleotidsequenz", daß sich die Nukleotidsequenz von Adaptoren aus verschiedenen Gruppen (oder auch innerhalb einer Gruppe) soweit unterscheidet, daß keine Kreuzhybridisierung bei der in Schritt (f) des erfindungsgemäßen Verfahrens erfolgenden Auswertung der Sequenzierungsreaktion unter Verwendung verschiedener markierter Hybridisierungssonden erfolgt. Weitere Kriterien, die vorzugsweise bei der Auswahl der Adaptorsequenzen beachtet werden sollen, sind ein möglichst hoher Schmelzpunkt (GC-Gehalt), das Fehlen von internen Haarnadelstrukturen und das Vorliegen von "seltenen" Sequenzen, die nur mit geringer Wahrscheinlichkeit in den zu sequenzierenden DNA-Fragmenten auftreten (z.B. 5'-CG-3' bei eukaryontischer DNA). Weiterhin ist es bevorzugt, wenn die verwendeten Adaptorsequenzen einen möglichst einheitlichen Schmelzpunkt aufweisen, so daß die Sequenzierung besser automatisierbar ist.

Man kann für die Ligationsreaktion innerhalb jeder Gruppe einen einzigen Adaptor oder zwei Adaptoren mit unterschiedlicher Nukleotidsequenz oder/und unterschiedlichen zweiten Enden verwenden, wobei die letztere Möglichkeit bevorzugt ist. Verwendet man unterschiedliche Adaptoren mit unterschiedlichen zweiten Enden, muß zur Klonierung der mit Adaptoren versehenen DNA-Fragmente folglich ein Basisvektor verwendet werden, der an zwei unterschiedlichen, zu den jeweiligen Enden der Adaptoren passenden Restriktionsstellen geöffnet wurde, d.h. unsymmetrisch hydrolysiert worden ist. Die Restriktionsstellen zur Einklonierung des DNA-Fragments sind beliebig, sofern sie bei Spaltung mit dem jeweiligen Enzym passende (und vorzugsweise nicht kompatible) Enden ergeben. Beispiele hierfür sind EcoRI-, PstI-, HindIII-Stellen etc.. Ferner ist es natürlich zweckmäßig, daß es sich bei den zur Klonierung verwendeten Restriktionsstellen auf dem Basisvektor um singuläre Stellen handelt.

Vorzugsweise weist der Basisvektor auf beiden Seiten der Insertionsstelle mindestens eine sogenannte "selten vorkommende enzymatische Spaltstelle auf. Unter einer selten vorkommenden Spaltstelle ist eine Spaltstelle zu verstehen, von der aufgrund ihrer spezifischen Charakteristiken zu erwarten ist, daß sie innerhalb der zu sequenzierenden DNA nur mit einer sehr geringen Wahrscheinlichkeit vorkommt. Beispiele für derartige Spaltstellen sind etwa Restriktionsstellen mit einer Erkennungssequenz von mindestens 7 Nukleotiden (z.B. NotI, SfiI, RsrII-, SgrAI-, SwaI-, PacI-, AscI-, PmeI-, Sse8387I-, SrsI- oder I-SceI-Stellen) oder/und solche Restriktionsstellen, die innerhalb ihrer Erkennungssequenz mindestens einmal die Nukleotidfolge 5'-CG-3' enthalten, die in eukaryontischer DNA extrem selten vorkommt. Diese selten vorkommende Spaltstelle kann zum Herausschneiden der einklonierten DNA aus dem Vektor ohne eine Spaltung innerhalb der DNA verwendet werden.

Weiterhin ist bevorzugt, aber nicht notwendig, daß man zur Klonierung der mit Adaptoren versehenen DNA-Fragmente einen Basisvektor verwendet, der in Nachbarschaft der zur Insertion der DNA-Fragmente verwendeten Restriktionsstellen Transkriptionsterminatoren aufweist, so daß keine negativen Wechselwirkungen des Vektors mit der Wirtszelle aufgrund einer möglichen Transkription der einklonierten DNA-Sequenzen auftreten können. Bevorzugte Beispiele für geeignete Basisvektoren sind etwa solche, die eine "multiple cloning site" mit entsprechenden geeigneten singulären Restriktionsstellen zur Klonierung aufweisen. Beispiele hierfür sind insbesondere die kommerziell erhältlichen Dideoxy-Sequenzierungsvektoren, die in Tabelle 7.1.1 von Ausubel et al., Current Protocols in Molecular Biology, Supplement 16 (Kapitel 7.1.3) angegeben sind. Aus diesen Ausführungen ist ersichtlich, daß die Voraussetzungen zur Verwendbarkeit eines Vektors für das erfindungsgemäße Verfahren weitaus geringer sind als für die Verwendbarkeit eines Vektors bei dem bekannten Multiplex-Verfahren.

Schritt (e) des erfindungsgemäßen Verfahrens umfaßt die Auswahl einer Anzahl unterschiedlicher Klone aus (d), wobei jedoch aus einer bestimmten Gruppe nur maximal ein Klon stammen darf und die Durchführung von Sequenzierungsreaktionen an diesen ausgewählten Klonen. Die Bereitstellung dieser Klone kann beispielsweise dadurch erfolgen, daß nach dem Klonierungsschritt gemäß (d) eine Transformation der ligierten DNA in geeignete Wirtszellen (vorzugsweise E.coli-Zellen) eine Vermehrung der auf diese Weise erhaltenen Genbank und eine Überprüfung einzelner Klone aus dieser Genbank auf das Vorhandensein insertierter DNA-Vektor nach bekannten Methoden (z.B. präparative Gelelektrophorese und Isolierung der Plasmid-DNA) erfolgen kann.

Die Sequenzierungsreaktion an den ausgewählten Klonen erfolgt prinzipiell wie bei dem bereits bekannten Multiplex-Sequenzierungsverfahren entweder nach einer chemischen Methode oder nach einer enzymatischen Kettenabbruchmethode. Dabei werden die einzelnen basenspezifischen Reaktionen (entweder nach der Sanger-Technik oder nach der Maxam-Gilbert-Technik) vorzugsweise direkt an der Mischung verschiedener DNA-Fragmente, d.h. simultan durchgeführt.

Bei einer chemischen Methode nach Maxam-Gilbert müssen zunächst die zu sequenzierenden DNA-Fragmente einschließlich der Adaptoren aus dem Vektor herausgeschnitten werden. Dies kann beispielsweise an den Restriktionsstellen erfolgen, an denen auch die Klonierung in den Vektor erfolgt ist. Auf diese Weise werden die in Schritt (d) einklonierten DNA-Fragmente wieder freigesetzt. Vorzugsweise werden zu diesem Zweck jedoch "selten vorkommende" Spaltstellen verwendet, die sich in Nachbarschaft, vorzugsweise in einer Entfernung von weniger als 100 Nukleotiden von der Klonierungsstelle befinden. Auf diese Weise ist die Wahrscheinlichkeit einer Spaltung innerhalb der zu sequenzierenden DNA nur äußerst gering. Die herausgeschnittenen Fragmente werden anschließend von Vektor-DNA gereinigt. Dies erfolgt vorzugsweise durch eine präparative Agarose-Gelelektrophorese. So erhält man ein Gemisch von DNA-Fragmenten, wobei jedes DNA-Fragment im Bereich seiner beiden Enden Adaptormoleküle aufweist, die von den Adaptormolekülen eines anderen DNA-Fragments verschieden sind. Dieses Gemisch wird anschließend nach einem bekannten Maxam-Gilbert-Protokoll behandelt. Dies bedeutet im allgemeinen ein Aufteilen des Gemisches in vier Aliquots, wobei jedes Aliquot mit einem unterschiedlichen, basenspezifischen, chemischen Reagenz behandelt wird, was schließlich zu einer statistischen Spaltung der DNA-Fragmente an den jeweiligen Basen führt. Somit wird eine Population von Sequenzierungsprodukten erzeugt, deren Sequenz in einem nächsten Schritt bestimmt werden kann.

Bei einer enzymatischen Kettenabbruchmethode nach Sanger ist ein Herausschneiden der DNA-Fragmente aus dem Vektor nicht erforderlich. Zunächst wird das Gemisch der Vektoren mit unterschiedlichen einklonierten DNA-Fragmenten denaturiert. Zu der resultierenden einzelsträngigen DNA wird ein Primer-Oligonukleotid zugegeben, das mit einem Bereich des Vektors in Nachbarschaft des einklonierten DNA-Fragments oder/und mit dem Adaptor komplementär ist, und daher an die denaturierte DNA bindet. Wenn die Sequenzierung des DNA-Fragments von zwei Seiten erfolgen soll, so können zu jedem Ansatz zwei unterschiedliche Primermoleküle zugegeben werden. Nach Aufteilung des so erhaltenen Gemisches in vier Aliquots wird nach dem bekannten Sanger-Protokoll eine Polymerisationsreaktion mit einer DNA-Polymerase jeweils in Gegenwart eines unterschiedlichen Kettenabbruchmoleküls durchgeführt. Die auf diese Weise erzeugte Population von Sequenzierungsprodukten wird anschließend wie im folgenden beschrieben weiterbehandelt.

Schritt (f) des erfindungsgemäßen Verfahrens, die Auswertung der Sequenzierungsreaktion unter Verwendung von nachweisbaren Hybridisierungssonden, die jeweils nur für einen einzigen Klon aus der Gesamtzahl von Klonen spezifisch sind, umfaßt im allgemeinen die folgenden Schritte:
(f1) Auftrennen der durch die Sequenzierungsreaktionen aus (e) erhaltenen Sequenzierungsprodukte nach ihrer Größe, wobei entsprechende Sequenzierungsprodukte einer bestimmten Reaktion aus mehreren Klonen gemeinsam aufgetrennt werden,
(f2) Transferieren der aufgetrennten Sequenzierungsprodukte auf einen zur Bindung von Nukleinsäuren geeigneten Träger und gegebenenfalls Immobilisieren der transferierten Sequenzierungsprodukte auf dem Träger,
(f3) reversibles Hybridisieren des Trägers mit (i) einer ersten nachweisbaren Hybridisierungssonde oder (ii) mit zwei oder mehreren, selektiv nachweisbaren Hybridisierungssonden, wobei jede Sonde jeweils nur für einen einzigen Klon aus (e) spezifisch ist und wobei die Spezifität jeder Sonde durch eine bei den Reaktionsbedingungen erfolgende Hybridisierung an eine bestimmte Zielsequenz definiert wird, die aus einem bestimmten Adaptor und gegebenenfalls benachbarten Vektorsequenzen gebildet wird,
(f4) Auswerten der Ergebnisse aus (f3), Entfernen der gebundenen Hybridisierungssonden vom Träger und
(f5) gegebenenfalls Wiederholen der Schritte (f3) und (f4) mit weiteren nachweisbaren Hybridisierungssonden, die sich von den anderen Sonden dadurch unterscheiden, daß sie an andere Zielsequenzen binden.

Die Auftrennung der Sequenzierungsprodukte (f1) erfolgt vorzugsweise durch Gelelektrophorese, besonders bevorzugt durch eine denaturierende Polyacrylamid-Gelelektrophorese in einem speziellen Sequenzgel. Als Träger, auf den die aufgetrennten Sequenzierungsprodukte transferiert werden (f2), verwendet man vorzugsweise eine Membran aus Nitrocellulose, Nylon, Polyvinylidenfluorid oder chemisch aktivierter Cellulose (z.B. Diazocellulose). Die Übertragung der DNA-Fragmente auf den Träger und ihre Immobilisierung erfolgt mit Hilfe bekannter Blotting-Techniken (siehe z.B. Sambrook et al., A Laboratory Manual, Cold Spring Harbor Laboratory Press, 1989).

Anschließend erfolgt eine reversible Hybridisierung des Trägers mit einer ersten nachweisbaren Hybridisierungssonde, die jeweils nur für einen einzigen Klon spezifisch ist. Es können aber auch zwei oder mehrere Hybridisierungssonden gleichzeitig verwendet werden, vorausgesetzt, sie sind nebeneinander selektiv aufgrund einer unterschiedlichen Markierung nachweisbar. Die Spezifität einer Sonde wird dabei alleine durch den gruppen- bzw. klonspezifischen Adaptor bestimmt. Die Hybridisierung der Sonde kann aber bei Verwendung kurzer Adaptoren nicht nur an Adaptor selbst, sondern auch zusätzlich noch an benachbarte Vektorsequenzen erfolgen. Die Länge des Hybridisierungsbereichs zwischen der Sonde und der spezifischen Zielsequenz beträgt vorzugsweise 10 bis 50 Nukleotide, besonders bevorzugt 15 bis 40 Nukleotide. Auf diese Weise wird eine die Bestimmung der Nukleinsäuresequenz erlaubende Hybridisierung erzielt.

Die Entfernung einer reversibel an die komplementäre Zielsequenz hybridisierten Sonde vom Träger erfolgt ebenfalls nach bekannten Methoden, z.B. durch Erhitzen über den Schmelzpunkt des Hybrids in Gegenwart von SDS (siehe z.B. Sambrook et al., supra).

Für das erfindungsgemäße Verfahren kann man sowohl radioaktiv markierte als auch nicht radioaktiv markierte Hybridisierungssonden. Vorzugsweise verwendet man nicht-radioaktiv markierte Sonden wie etwa Biotin-, Fluoreszenz-, Lumineszenz-, Digoxigenin- oder/und Enzym- (z.B. Peroxidase oder alkalische Phosphatase) markierte Sonden. Besonders bevorzugt ist die Verwendung von Sonden, die mit Digoxigenin oder Derivaten davon markiert sind. Erfindungsgemäße Sonden können auf einfache Weise durch eine chemische Synthese erzeugt werden, wobei der Einbau von markierten Nukleotiden während der Synthese erfolgen kann. Andererseits kann die Sonde auch nachträglich z.B. durch 5'-Phosphorylierung radioaktiv markiert werden.

In der beigefügten Zeichnung wird eine besonders bevorzugte Ausführungsform des erfindungsgemäßen Verfahrens zur simultanen Sequenzierung nochmals schematisch dargestellt. Die als Ausgangsmaterial benötigten komponenten sind ein Vektor (A) und zwei Adaptormoleküle (B) und (C), die aus doppelsträngiger DNA bestehen und die jeweils an einer Seite mit einem glatten und an der anderen Seite mit einem überstehenden Ende versehen sind.

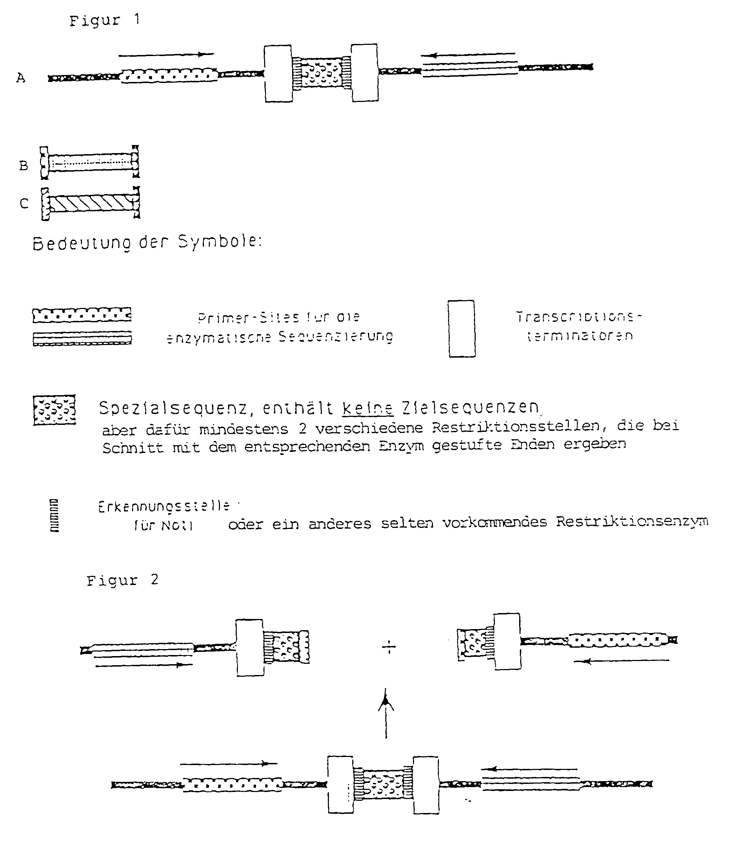

Figur 1 zeigt die Ausgangsmaterialien.

Der Vektor wird mit zwei verschiedenen Restriktionsenzymen hydrolysiert, die jeweils überhängende, nicht kompatible Enden erzeugen.

Figur 2 stellt diese Hydrolyse dar.

Die Sequenzier-DNA mit glatten Enden (D) wird an die Adaptormoleküle legiert und in den geöffneten Vektor einkloniert. Vorzugsweise wird dazu die erhaltene Sequenzier-DNA durch Agarosegelelektrophorese aufgetrennt und ein gewünschter Größenbereich, vorzugsweise 800 bp bis 1200 bp aus dem Gel ausgeschnitten und eluiert.

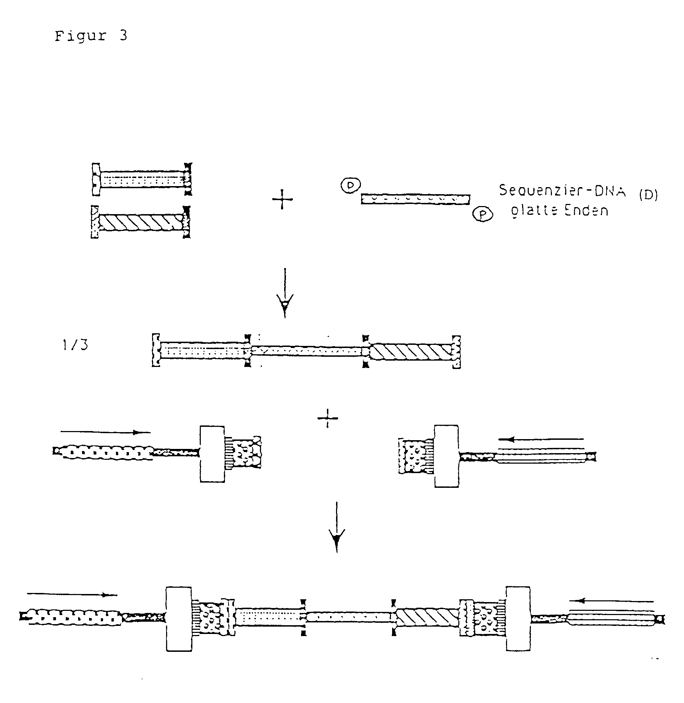

Figur 3 erläutert dies schematisch.

Der auf diese Weise erhaltene Vektor mit insertierter DNA kann durch Transformation in geeignete Zellen (im allgemeinen E.coli-Zellen) vermehrt und isoliert werden.

Ein weiterer Gegenstand der vorliegenden Erfindung ist ein Verfahren zur Umwandlung einer bereits vorhandenen Genbank in eine modifizierte Genbank, an der eine simultane Sequenzierung von Nukleinsäuren (wie oben beschrieben) durchführbar ist. Der Begriff "Genbank" bedeutet hier, daß eine Anzahl unterschiedlicher (zu sequenzierender) DNA-Fragmente als Insertionen in einem Basisvektor vorliegt. Das Verfahren zur Modifizierung einer Genbank beruht darauf, daß ein doppelsträngiges DNA-Adaptormolekül in Nachbarschaft der zu bestimmenden Insertionen in den Vektor eingeführt wird, wodurch bei Verwendung mehrerer unterschiedlicher Adaptormoleküle die Genbank in eine Anzahl von Gruppen aufgeteilt werden kann, an denen eine simultane Sequenzierung durchgeführt werden kann.

Dieses Verfahren umfaßt die Schritte:
(a) Bereitstellen einer beliebigen Genbank, bestehend aus einer Vielzahl von unterschiedlichen DNA-Fragmenten, die in einen Basisvektor kloniert worden sind, wobei der Basisvektor in Nachbarschaft der einklonierten DNA-Fragmente mindestens eine singuläre, selten vorkommende enzymatische Spaltstelle enthält,
(b) Linearisieren der Genbank durch Spaltung an einer selten vorkommenden Spaltstelle gemäß (a),
(c) Aufteilen der Genbank in mehrere Gruppen,
(d) Ligieren der aufgeteilten, linearisierten Genbank mit einem doppelsträngigen DNA-Adaptor, wobei der doppelsträngige Bereich des Adaptors eine Länge von ≥ 5 Nukleotiden besitzt und an beiden Seiten Enden aufweist, die zur Spaltstelle des Basisvektors passend sind, wobei an jede Gruppe der Genbank ein Adaptor mit unterschiedlicher Sequenz ligiert wird, und
(e) gegebenenfalls Abtrennen der gewünschten Ligationsprodukte von Nebenprodukten.

Dabei wird vorzugsweise die Nukleotidsequenz des Adaptormoleküls so gewählt, daß durch die Einführung des Adaptors die Spaltstelle im Vektor eliminiert wird. Auf diese Weise können die gewünschten Ligationsprodukte von Nebenprodukten abgetrennt werden, indem sie mit dem in Schritt (b) zum Linearisieren der Genbank verwendeten Restriktionsenzym nachgespalten werden. Auf diese Weise wird ein religierter Vektor (ohne Adaptormolekülinsertion) wieder geöffnet, so daß der Vektor mit einklonierter DNA als einziges transformierbares Produkt erhalten wird. Die "selten vorkommende Spaltstelle" bei diesem Verfahren ist wie oben definiert, d.h. sie hat vorzugsweise eine Erkennungssequenz von mindestens 8 Nukleotiden Länge oder/und mindestens einmal die Nukleotidsequenz 5'-CG-3', z.B. eine NotI-, SfiI-, RsrII, SgrAI, SwaI, PacI, AscI, PmeI, Sse8387I, SrsI oder I-SceI-Erkennungsstelle.

Im folgenden wird eine besonders bevorzugte Ausführungsform für die Umwandlung von konventionellen Genbanken in Genbanken, an denen eine simultane DNA-Sequenzierung möglich ist, schematisch dargestellt. Dabei geht man von einer Genbank in einem Basisvektor (A) aus, der eine selten vorkommende Restriktionsstelle (z.B. NotI) in Nachbarschaft der Insertion aufweist. Als Basisvektoren werden vorzugsweise moderne Hochleistungsvektoren verwendet (z.B. pBsSK-Vektoren). Weiterhin benötigt man als Ausgangsmaterial einen Satz spezieller Adaptor-Oligonukleotide (B), die für die Restriktionsstelle kompatible Enden aufweisen. Die Adaptormoleküle sind vorzugsweise dephosphoryliert und die Sequenzen neben den überhängenden Bereichen am Ende sollen vorzugsweise nicht der NotI-Erkennungssequenz entsprechen. Der doppelsträngige Bereich der Adaptormoleküle enthält die zur simultanen DNA-Sequenzierung benötigten Zielsequenzen (an die später die Hybridisierungssonde bilden soll). Deshalb wird eine entsprechend hohe Anzahl von Adaptormolekülen mit "unterschiedlicher Nukleotidsequenz" (siehe obige Definition) benötigt. Insbesondere darf zwischen den einzelnen Adaptormolekülen keine die Sequenzbestimmung störende Kreuzhybridisierung auftreten.

In einem ersten Schritt wird der Vektor an einer selten vorkommenden Restriktionsstelle (hier NotI) in Nachbarschaft (vorzugsweise ≤ 100 Nukleotide Entfernung) von der DNA-Insertion hydrolysiert und in mehrere Gruppen aufgeteilt. Anschließend wird jede Gruppe des hydrolysierten Vektors mit einem unterschiedlichen dem doppelsträngigen Adaptormolekül ligiert.

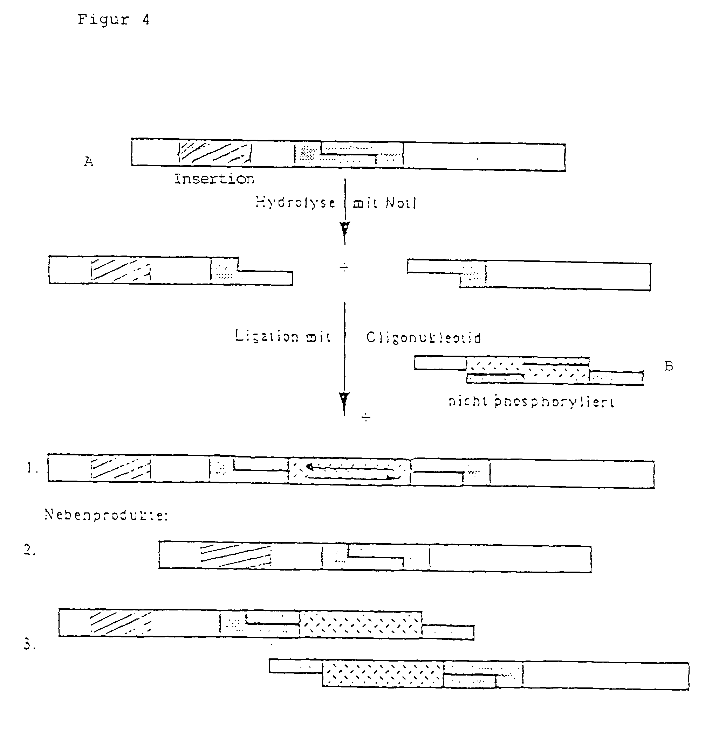

Bei diesem Prozeß entstehen neben dem gewünschten Produkt (1) noch unerwünschte Nebenprodukte (2) und (3), von denen (3) nicht transformierbar ist (und daher keine spezielle Abtrennung erfordert) und (2) durch Nachspaltung mit NotI wieder linearisiert und damit untransformierbar gemacht werden kann (wenn die Nukleotidsequenz des Adaptormoleküls so gewählt ist, daß die NotI-Stelle beseitigt wird). Dies zeigt Figur 4 schematisch.

Die Sequenzierungsreaktion an einer derartigen Genbank erfolgt nach dem bereits oben beschriebenen Prinzip.

Bei der erfindungsgemäßen Modifizierung einer Genbank kann man einerseits ein doppelsträngiges Adaptormolekül verwenden, das eine symmetrische Sequenz aufweist und aus zwei identischen selbst-komplementären Oligonukleotiden zusammengesetzt ist, aber andererseits auch ein solches Adaptormolekül verwenden, das eine unsymmetrische Sequenz aufweist. Die Verwendung eines unsymmetrischen Adaptormoleküls ist bevorzugt. In diesem Fall muß für die Auswertung einer Sequenzreaktion ein Gemisch von 2 Hybridisierungssonden (die jeweils zu einem Strang des Adaptormoleküls komplementär, aber nicht selbstkomplementär sind) eingesetzt werden.

Wenn man als Ausgangsprodukt für das Verfahren eine Genbank in einem Basisvektor verwendet, die auf beiden Seiten in Nachbarschaft der einklonierten, zu sequenzierenden DNA-Fragmente jeweils mindestens eine singuläre, selten vorkommende Restriktionsstelle enthält, so können die Schritte (b) bis (e) des erfindungsgemäßen Verfahrens zweimal durchgeführt werden. Auf diese Weise befindet sich dann auf beiden Seiten des einklonierten DNA-Fragments ein spezifisches Adaptormolekül, das bei einem simultanen Sequenzierungsprozeß, wie oben beschrieben, jeweils eine spezifische Hybridisierungssonde binden kann, so daß eine Sequenzierung des einklonierten DNA-Fragments, ausgehend von beiden Enden des Fragments, möglich ist.

Weiterhin soll die Erfindung durch die folgenden Sequenzprotokolle und Beispiele verdeutlicht werden.
- SEQ ID NO: 1: Adaptor (1)
- SEQ ID NO: 2: Adaptor (2) Im Sequenzprotokoll ist der komplementäre Strang in 5'-3'-Richtung des eingesetzten Adaptors aufgeführt.
- SEQ ID NO:: 3 Adaptor (3)
- SEQ ID NO: 4: Adaptor (4) Im Sequenzprotokoll ist der komplementäre Strang in 5'-3'-Richtung des eingesetzten Adaptors aufgeführt.
- SEQ ID NO: 5: Multiplexvektor 10 - Plex 10E
- SEQ ID NO: 6: Multiplexvektor 10 - Plex 10P
- SEQ ID NO: 7: Multiplexvektor 19 - Plex 19E
- SEQ ID NO: 8: Multiplexvektor 19 - Plex 19P
- SEQ ID NO: 9: Adaptor GB1 Die Adaptor-DNA besteht in 5'-3'-Richtung aus den Basen 1-23 und in 3'-5'-Richtung aus den zu 5-27 komplementären Basen.
- SEQ ID NO: 10: Adaptor GB2 Die Adaptor-DNA besteht in 5'-3'-Richtung aus den Basen 1-22 und in 3'-5'-Richtung aus den zu 5-26 komplementären Basen.
- SEQ ID NO: 11: Oligonukleotid BG1A
- SEQ ID NO: 12: Oligonukleotid BG1B
- SEQ ID NO: 13: Oligonukleotid BG2A
- SEQ ID NO: 14: Oligonukleotid BG2B
- SEQ ID NO: 15: T3-Sequenzierungsprimer

### Beispiele

### Beispiel 1:

### Anlegen einer Genbank nach dem Multiplex-Verfahren Fragmentieren der DNA:

10 µg chromosomale Hefe-DNA (gelöst in 50 µl 50 mmol/l Tris-HCl, 10 mmol/l MgCl₂, 10 mmol/l DTT pH 7,8) wurden sclange in einem Ultraschallbad (Sonorex RK255H, BANDELIN, Frequenz: 35 kHz; Leistung: 160/320 W) beschallt, bis die erhaltenen Fragmente bei Analyse mittels Agarosegel-Elektrophorese kleiner als 5 kBp waren.

Glätten der Enden und Auftrennung nach Größe: Zum Glätten der Enden wurden die DNA-Fragmente 30 min. lang in einem Volumen von 40 µl mit Klenow-Polymerase (0,25 U/µl) und T4-DNA-Polymerase (0,15 U/µl) in Gegenwart von dNTPs (0,025 mmol/l) umgesetzt. Nach Inaktivieren dieser Enzyme (10 min. Erhitzen bei 70 °C) wurde ein Teil dieser DNA auf ein Agarosegel (0,8 % low melting point Agarose, 1 x TBE) aufgetragen und durch Elektrophorese (1 h bei 4 V/cm) aufgetrennt.

### Isolieren der ca. 1 kBp langen Fragmente:

Der Bereich des Gels, der zwischen 0,5 und 1,1 kBp lange Fragmente enthält, wurde aus dem Gel ausgeschnitten. Die DNA-Fragmente wurden durch Einfrieren der Agarose und anschliessende Zentrifugation eluiert und durch Zugabe von 1/10 Volumen Natriumacetat (3 mol/l, pH 7) und 0,8 Volumen Isopropanol gefällt. Die gefällte DNA wurde mit 70 % EtOH(V/V) gewaschen, getrocknet und in TE (10 mmol/l Tris-HCl, 0,1 mmol/l EDTA pH 8) aufgenommen.

### Klonieren der Fragmente in den Multiplex-Vektor 10 oder 19

Etwa 20 ng der erhaltenen Fragmente wurden mit Hilfe der T4 DNA-Ligase (0,3 Weiss Einheiten/µl) in einem Volumen von 20 µl (50 mmol/l Tris-HCl, 10 mmol/l MgCl₂, 10 mmol/l DTT, pH 7,8, 0,5 mmol/l rATP) mit 20 ng des Multiplex-Vektors 10 oder 19 (entspricht den Vektoren 1 oder 3 des Multiplex-Kits der Fa. Millipore) ligiert (22 h bei 16°C). Der Multiplex-Vektor war zuvor unter Standardbedingungen mit SmaI hydrolisiert und anschließend dephosphoryliert worden.

### Transformieren und Kultivieren von DH5a-Zellen

Nach den Angaben der Fa. BRL wurden käuflich erworbene kompetente DH5a-Zellen mit einem Zehntel des Ligationsansatzes transformiert und unter Selektion auf Tetracyclin kultiviert. Erhalten wurden - bezogen auf den eingesetzten Vektor - 5 x 10⁵ unabhängige Kolonien pro µg DNA.

### Anlegen von Genbanken, die ausschließlich Plasmide mit klonierter Hefe-DNA enthalten

Eine LB-Platte mit etwa 1000 der erhaltenen Kolonien wird so lange bei 37°C kultiviert, bis die Kolonien einen Durchmesser von ca. 1 bis 2 mm erreicht haben. Dann werden die Kolonien mit Hilfe eines Glasspatels in LB-Medium suspendiert, von der Platte abgenommen und unter Selektion auf Tetrazyklin in einem Volumen von 3 ml für weitere 2 Stunden kultiviert. Danach werden die Bakterien durch Zentrifugation sedimentiert. Die Plasmid-DNA wird durch alkalische Lyse der Zellen unter Standardbedingungen isoliert und durch Säulenchromatographie (Qiagen, Minipreparation) gereinigt. Ein Teil dieser DNA (ca. 2 µq) wird auf ein Agarosegel (0,8 % low melting point Agarose, 1 x TBE, 0,5 mg/ml Ethidiumbromid) aufgetragen und durch Elektrophorese (1 h bei 4 V/cm) aufgetrennt. Der Bereich des Gels zwischen der "supercoiled form" und der "relaxed form" des Multiplexvektors 10 oder 19 enthält die Plasmid-DNA mit klonierter Hefe-DNA. Dieser Bereich des Gels wird ausgeschnitten. Die darin enthaltene DNA wird durch Einfrieren der Agarose und anschließende Zentrifugation eluiert und durch Zugabe von 1/10 Volumen Natriumacetat (3 mol/l, pH 7) und 0,8 Volumen Isopropanol gefällt. Die gefällte DNA wird mit 70 % EtOH (V/V) gewaschen, getrocknet und in TE (10 mmol/l Tris-HCl, 0,1 mmol/l EDTA, pH 8) aufgenommen. Mit einem Teil der erhaltenen DNA werden kompetente DH5α-Zellen transformiert und unter Selektion auf Tetracyclin auf LB-Platten kultiviert.

### Beispiel 2:

### Anlegen einer Genbank nach dem beschriebenen Verfahren

Chromosomale DNA der Hefe wurde - wie unter Beispiel 1 beschrieben - durch Beschallen mit Ultraschall in Fragmente zertrümmert, die kleiner als 5 kBp sind. Die Enden dieser DNA-Fragmente wurden durch Behandlung mit Klenow-Polymerase und T4-DNA-Polymerase geglättet. Nach Inaktivierung dieser Enzyme (10 min. bei 70°C) wurden 2 µg dieser DNA in einem Volumen von 20 µl (50 mmol/l Tris-HCl, 10 mmol/l MgCl₂, 10 mmol/l DTT pH 7,8, 1 mmol/l rATP) mit T4DNA-Ligase (50 Weiss Einheiten pro ml) und 2 µg von jedem der folgenden AdaptorMoleküle umgesetzt:

Die Aufarbeitung der so erhaltenen DNA-Fragmente erfolgt wie unter Beispiel 1 angegeben: Nach der Ligation wurden die DNA-Fragmente auf ein 0,8 % Agarosegel aufgetragen und aufgetrennt. Die Fragemente, die zwischen 0,5 und 1,1 kBp lang waren, wurden eluiert und gereinigt. Danach wurden sie in den Multiplex-Vektor 10 oder 19 kloniert, der im Gegensatz zu Beispiel 1 durch Behandlung mit EcoRI und PstI zuvor entsprechend abgestufte, nicht zueinander kompatible Enden besaß. Mit dem Ligationssatz wurden kompetente DH5a-Zellen transformiert und unter Selektion auf Tetracyclin auf LB-Platten kultiviert.
Erhalten wurden - bezogen auf den eingesetzten Vektor - 3 x 10⁶ unabhängige Kolonien pro µq DNA und damit etwa 6 mal mehr Kolonien als im Beispiel 1.

Genbanken, die ausschließlich Plasmide mit klonierter Hefe-DNA enthalten, wurden wie in Beispiel 1 beschrieben angelegt.

### Beispiel 3:

### Simultane Sequenzierung von Genbanken, die nach dem Multiplex-Verfahren oder nach dem neuen Verfahren angelegt wurden.

Durch Ausstreichen von Genbanken, die entsprechend dem Beispiel 1 und dem Beispiel 2 erhalten wurden, wurden Einzelkolonien angelegt. In 10 ml LB-Medium wurden jeweils 1 Kolonie von beiden Genbanken vereinigt und suspendiert. Das Medium wurde solange bei 37°C geschüttelt, bis die Suspension eine optische Dichte von 2 bei einer Wellenlänge von 600 nm aufwies. Danach wurden die Bakterien durch Zentrifugation sedimentiert. Die Plasmid-DNA wurde durch alkalische Lyse der Zellen unter Standardbedingungen isoliert und durch Säulenchromatographie (Qiagen, Minipräparation) gereinigt.

Das Gemisch der beiden Plasmid-DNAs (8 µg) wurde unter Verwendung einer modifizierten T7-Polymerase (Sequenase) und den Protokollen der Fa. USB sequenziert. Als Sequenzierungsprimer wurde ein Gemisch der Oligonukleotide Plex E und Plex P (jeweils 40 ng pro Ansatz) verwendet, das standardmäßig zur Sequenzierung nach dem Multiplex-Verfahren verwendet wird. Die erhaltenen Sequenzprodukte wurden auf einem Sequenzgel (40 cm lang, 0,4 mm dick, 6 % Polyacrylamid, 7 mol/l Harnstoff; Puffer: 1 x TBE) aufgetrennt, durch einen Kapillarblott auf eine neutrale Nylonmembran übertragen und durch Bestrahlen mit 256 nm UV-Licht auf der Membran fixiert. Die Sequenzprodukte wurden durch Hybridisierung mit digoxigenierten Oligonukleotiden nachgewiesen, die durch Umsetzung der unmodifizierten Oligonukleotide mit terminaler Deoxynukleotidyltransferase und digoxigeniertem dUTP (Boehringer Mannheim) erhalten worden waren. Die entsprechenden Sequenzleitern wurden mit Hilfe des Digoxigenin-Nachweissystems der Fa. Boehringer Mannheim (Fab-Fragmente von anti-Digoxigenin Antikörpern) und anschließender Chemolumineszenz sichtbar gemacht. Nach dem Nachweis einer Sequenzreaktion wurde das hybridisierte Oligonukleotid durch Erhitzen der Membran unter Standardbedingungen entfernt. Die Membran wurde jeweils einmal mit einem der folgenden digoxigenierten Oligonukleotide umgesetzt:

### Ergebnis:

Bei jeder der durchgeführten Hybridisierungen konnte eine unabhängige eindeutige Sequenz bestimmt werden.

### Beispiel 4:

### Umwandeln einer bereits vorhandenen Genbank in eine Genbank die zur simultanen Sequenzierung geeignet ist.

### Isolierung der cDNA-haltigen Plasmide:

Eine cDNA-Bank aus menschlichem Herz, angelegt in der EcoRI-Schnittstelle des Phagen-Vektors Lambda ZAP II und bei der Fa. Stratagene (San Diego, USA) käuflich zu erhalten (Katalog Nr. 936208), wurde zunächst - nach den Angaben des Herstellers - in die doppelsträngige Phagemidform überführt.

### Hydrolyse mit NotI und Anheften der Adaptoren:

1 µg der aus der Phagemid-Bank isolierten Gesamt-DNA wurde nach Standardbedingungen mit dem Enzym NotI gespalten. In zwei getrennten Reaktionen wurden zu 500 ng der gespaltenen DNA jeweils 300 ng der Adaptor-DNA (Ansatz 1: Adaptor GB1; Ansatz 2: Adaptor GB2) zugegeben. Die Adaptoren wurden durch Umsetzung mit T4-DNA-Ligase (50 Weiss-Einheiten/ml) in einem Volumen von 20 µl (50 mM Tris-HCl, 10 mM MgCl₂, 10 mM DTT, pH 7,8, 1 mM ATP) mit der DNA ligiert (10 h; 12°C). Die eingesetzten Adaptoren haben folgende Sequenz:

### Abtrennen der überschüssigen Adaptoren

Nach der Ligation wird das Reaktionsgemisch 10 min auf 65°C erhitzt und anschließend auf Eis abgekühlt. Die Reaktionsansätze werden auf ein Agarosegel (0,8 % low melting point Agarose, 1 x TBE, 0,5 mg/ml Ethidiumbromid) aufgetragen und durch Elektrophorese (1 h bei 4 V/cm) aufgetrennt. Der Bereich des Gels mit Plasmid-DNA (größer als linearisierte pBsSKII-DNA) wird ausgeschnitten. Die darin enthaltene DNA wird durch Einfrieren der Agarose und anschließende Zentrifugation eluiert und durch Zugabe von 1/10 Volumen Natriumacetat (3 mol/l, pH 7) und 0,8 Volumen Isopropanol gefällt. Die gefällte DNA wird mit 70% EtOH (V/V) gewaschen, getrocknet und in TE (10 mmol/l Tris-HCl, 0,1 mmol/l EDTA, pH 8) aufgenommen.

### Zirkularisieren der Plasmid-DNA mit Adaptoren an beiden Enden durch Annealen der kohäsiven Enden

Ca. 50 ng der isolierten DNA werden in einem Volumen von 20 µl (50 mM Tris-HCl, 10 mM MgCl₂, 10 mM DTT, pH 7,8) zunächst für 20 min bei 65°C und dann weitere 2 h bei 37°C aufbewahrt.

### Anlegen von Genbanken

Nach den Angaben der Fa. BRL wurden käuflich erworbenen kompetenten DH5α-zellen mit einem Zehntel der Ligationsansätze transformiert. Die Bakterien werden auf LB-Platten ausgestrichen und unter Selektion auf Ampicillin kultiviert. Die so angelegten Genbanken enthalten - bezogen auf den eingesetzten Vektor - 1 bis 6 x 10⁶ unabhängige Kolonien pro µg an eingesetzter DNA.

Jeweils eine Kolonie von jeder der beiden Genbanken wurde abgenommen und zusammen in 10 ml LB-Medium kultiviert. Wie unter Beispiel 3 beschrieben, wurde Plasmid-DNA isoliert und das Gemisch der beiden Plasmid-DNAs (8 µg) unter Verwendung einer modifizierten T7-Polymerase (Sequenase) und den Protokollen der Fa. USB sequenziert. Als Sequenzierungsprimer wurde der T3-Sequenzierungsprimer (5'-ATTAACCCTCACTAAAG-3' (SEQ ID NO: 15), jeweils 40 ng pro Ansatz) verwendet. Die erhaltenen Sequenzprodukte wurden auf einem Sequenzgel (40 cm lang, 0,4 mm dick, 6 % Polyacrylamid, 7 mol/l Harnstoff; Puffer: 1 x TBE) aufgetrennt, durch einen Kapillarblot auf eine neutrale Nylonmembran übertragen und durch Bestrahlen mit 256 nm UV-Licht auf der Membran fixiert. Die Sequenzprodukte wurden durch Hybridisierung mit digoxigenierten Oligonukleotiden nachgewiesen, die durch Umsetzung der unmodifizierten Oligonukleotide mit terminaler Deoxynukleotidyltransferase und digoxigeniertem dUTP (Boehringer Mannheim) erhalten worden waren. Die entsprechenden Sequenzleitern werden mit Hilfe des Digoxigenin-Nachweissystems der Fa. Boehringer Mannheim (Fab-Fragmente von anti-Digoxigenin-Antikörpern) und anschließender Chemolumineszenz sichtbar gemacht. Nach dem Nachweis einer Sequenzreaktion wurde das hybridisierte Oligonukleotid durch Erhitzen der Membran unter Standardbedingungen entfernt.

Die Membran wird jeweils einmal mit einem Gemisch der Oligonukleotide BG1A und BG1B und anschließend mit einem Gemisch der Oligonukleotide BG2A und BG2B umgesetzt:

### Ergebnis:

Bei jeder der durchgeführten Hybridisierungen konnte eine unabhängige eindeutige Sequenz bestimmt werden.

### SEQUENZPROTOKOLL

(iii) ANZAHL DER SEQUENZEN: 15
(2) INFORMATION ZU SEQ ID NO: 1:
   (i) SEQUENZ CHARAKTERISTIKA:
      (A) LANGE: 24 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 1:
   AATTCCATAA CTGTAACCTT TAAC                24
(2) INFORMATION ZU SEQ ID NO: 2:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 2:
   CCATAACTGT AACCTTTAAC                     20
(2) INFORMATION ZU SEQ ID NO: 3:
   (i) SEQUENZ CHARAKTERISTIKA:
      (A) LANGE: 20 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 3:
   CTATTTGTAA TTCCGCTGCA 20
(2) INFORMATION ZU SEQ ID NO: 4:
   (i) SEQUENZ CHARAKTERISTIKA:
      (A) LÄNGE: 16 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 4:
   CTATTTGTAA TTCCGC 16
(2) INFORMATION ZU SEQ ID NO: 5:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 5:
   TATATATAGG GTATTAGGTG 20
(2) INFORMATION ZU SEQ ID NO: 6:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 6:
   TGAGTATATT GATGATTAGG 20
(2) INFORMATION ZU SEQ ID NO: 7:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 7:
   AGAAGTTAAT GTAGGGTTGG 20
(2) INFORMATION ZU SEQ ID NO: 8:
   (i) SEQUENZ CHARAKTERISTIKA:
      (A) LANGE: 20 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 8:
   GTGATAAGTA GAGTTGGTTG 20
(2) INFORMATION ZU SEQ ID NO: 9:
   (i) SEQUENZ CHARAKTERISTIKA:
      (A) LÄNGE: 27 Basenpaare
      (B) ART: Nukleinsäure
      (C) STRANGFORM: beides
      (D) TOPOLOGIE: linear
   (xi) SEQUENZBESCHREIBUNG: SEQ ID NO: 9:
   GGCCACATAA CTCAAATCTC AAAGGCC 27
(2) INFORMATION ZU SEQ ID NO: 10:
   (i) SEQUENZ CHARAKTERISTIKA:
      (A) LÄNGE: 26 Basenpaare
      (B) ART: Nukleinsäure
      (C) STRANGFORM: beides
      (D) TOPOLOGIE: linear
   (xi) SEQUENZBESCHREIBUNG: SEQ ID NO: 10:
   GGCCAGCTAT CTCGTAATTG CTGGCC 26
(2) INFORMATION ZU SEQ ID NO: 11:
   (i) SEQUENZ CHARAKTERISTIKA:
      (A) LANGE: 19 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 11:
   GAGTTATGTG GCCGCCACC 19
(2) INFORMATION ZU SEQ ID NO: 12:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 12:
   AGACGGCCAC ATAACTCAAA 20
(2) INFORMATION ZU SEQ ID NO: 13:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 13:
   CGAGATAGCT GGCCGCCA 18
(2) INFORMATION ZU SEQ ID NO: 14:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 14:
   AGAGCGGCCA GCTATCTC 18
(2) INFORMATION ZU SEQ ID NO: 15:
   (i) SEQUENZ CHARAKTERISTIKA:
      (A) LÄNGE: 17 Basenpaare
      (B) ART: Nukleinsäure
      (C) STRANGFORM: Einzel
      (D) TOPOLOGIE: linear
   (xi) SEQUENZBESCHREIBUNG: SEQ ID NO: 15:
   ATTAACCCTC ACTAAAG                                  17

## Patentansprüche

1. Verfahren zur simultanen Sequenzierung von Nukleinsäuren,
**gekennzeichnet durch**
(a) Bereitstellen einer Vielzahl DNA-Fragmente, deren Sequenz bestimmt werden soll,
(b) Aufteilen der Gesamtheit der zu sequenzierenden DNA-Fragmente in mehrere Gruppen,
(c) Ligieren von doppelsträngigen DNA-Adaptoren an beide Enden der aufgeteilten DNA-Fragmente aus (b), wobei die Adaptoren einen doppelsträngigen Bereich mit einer Länge von ≥ 5 Nukleotiden besitzen, ein erstes zu den Enden der DNA-Fragmente kompatibles Ende und ein zweites, zur Klonierung in einen Vektor geeignetes Ende aufweisen, und wobei an jede Gruppe der aufgeteilten DNA-Fragmente Adaptoren mit unterschiedlicher Nukleotidsequenz ligiert werden,
(d) Klonieren der an beiden Enden mit Adaptoren versehenen DNA-Fragmente aus (c) in einen Basisvektor, der zu den zweiten Enden der Adaptoren passende Restriktionsstellen aufweist,
(e) Auswählen einer Anzahl von Klonen aus (d), wobei jedoch aus einer Gruppe nur maximal 1 Klon stammen darf, und Durchführen von Sequenzierungsreaktionen an den ausgewählten Klonen, und
(f) Auswerten der Ergebnisse der Sequenzierungsreaktionen unter Verwendung einer Anzahl von nachweisbaren Hybridisierungssonden, die jeweils nur für einen einzigen Klon aus (e) spezifisch sind.

2. Verfahren nach Anspruch 1,
**dadurch gekennzeichnet,** daß die DNA-Fragmente glatte Enden aufweisen.

3. Verfahren nach Anspruch 1,
**dadurch gekennzeichnet,** daß die DNA-Fragmente gestufte Enden aufweisen.

4. Verfahren ach einem der Ansprüche 1 bis 3,
**dadurch gekennzeichnet,** daß man Adaptoren verwendet, deren zweites Ende gestuft ist.

5. Verfahren nach einem der Ansprüche 1 bis 4,
**dadurch gekennzeichnet,** daß die Länge der doppelsträngigen DNA-Adaptoren 10 bis 40 Nukleotide beträgt.

6. Verfahren nach einem der Ansprüche 1 bis 5,
**dadurch gekennzeichnet,** daß man innerhalb jeder Gruppe einen einzigen Adaptor verwendet.

7. Verfahren nach einem der Ansprüche 1 bis 6,
**dadurch gekennzeichnet,** daß man innerhalb jeder Gruppe zwei Adaptoren verwendet.

8. Verfahren nach einem der Ansprüche 1 bis 5 oder 7,
**dadurch gekennzeichnet,** daß man für die Ligationsreaktion innerhalb jeder Gruppe zwei Adaptoren mit unterschiedlicher Nukleotidsequenz und gestuften zweiten Enden verwendet und daß man zur Klonierung der mit Adaptoren versehenen DNA-Fragmente einen Basisvektor verwendet, der zwei unterschiedliche, zu den jeweiligen zweiten Enden der Adaptoren kompatible Restriktionsstellen aufweist.

9. Verfahren nach Anspruch 8,
**dadurch gekennzeichnet,** daß die gestuften zweiten Enden der beiden Adaptormoleküle nicht miteinander kompatibel sind.

10. Verfahren nach einem der Ansprüche 1 bis 9,
**dadurch gekennzeichnet,** daß der zur Klonierung der DNA-Fragmente verwendete Basisvektor zusätzlich an beiden Seiten der Insertionsstelle mindestens eine selten vorkommende enzymatische Spaltstelle aufweist.

11. Verfahren nach Anspruch 10,
**dadurch gekennzeichnet,** daß die selten vorkommende enzymatische Spaltstelle eine Erkennungssequenz von mindestens 7 Nukleotiden aufweist oder/und mindestens einmal die Nukleotidsequenz 5'-CG-3' enthält.

12. Verfahren nach Anspruch 11,
**dadurch gekennzeichnet,** daß die Spaltstelle eine NotI-, SfiI-, RsrII-, SgrAI-, SwaI-, PacI-, AscI-, PmeI-, Sse8387I-, SrsI- oder I-SceI-Schnittstelle ist.

13. Verfahren nach einem der Ansprüche 1 bis 12,
**dadurch gekennzeichnet,** daß man zur Klonierung der mit Adaptoren versehenen DNA-Fragmente einen Basisvektor verwendet, der in Nachbarschaft der zur Insertion der DNA-Fragmente verwendeten Restriktionsstellen Transkriptionsterminatoren aufweist.

14. Verfahren nach einem der Ansprüche 1 bis 13,
**dadurch gekennzeichnet,** daß man zur Auswertung der Sequenzierungsreaktionen nicht-radioaktiv markierte Hybridisierungssonden verwendet.

15. Verfahren nach Anspruch 14,
**dadurch gekennzeichnet,** daß man biotin-, fluoreszenz-, lumineszenz-, digoxigenin- oder/und enzym-markierte Hybridisierungssonden verwendet.

16. Verfahren nach Anspruch 1,
**dadurch gekennzeichnet,** daß (f) die folgenden Schritte umfaßt:
(f1) Auftrennen der durch die Sequenzierungsreaktionen aus (e) erhaltenen Sequenzierungsprodukte nach ihrer Größe, wobei entsprechende Sequenzierungsprodukte einer bestimmten Reaktion aus mehreren Klonen gemeinsam aufgetrennt werden,
(f2) Transferieren der aufgetrennten Sequenzierungsprodukte auf einen zur Bindung von Nukleinsäuren geeigneten Träger und gegebenenfalls Immobilisieren der transferierten Sequenzierungsprodukte auf dem Träger,
(f3) reversibles Hybridisieren des Trägers mit (i) einer ersten nachweisbaren Hybridisierungssonde oder (ii) mit zwei oder mehreren, selektiv nachweisbaren Hybridisierungssonden, wobei jede Sonde jeweils nur für einen einzigen Klon aus (e) spezifisch ist und wobei die Spezifität jeder Sonde durch eine bei den Reaktionsbedingungen erfolgende Hybridisierung an eine bestimmte Zielsequenz definiert wird, die aus einem bestimmten Adaptor und gegebenenfalls benachbarten Vektorsequenzen gebildet wird,
(f4) Auswerten der Ergebnisse aus (f3), Entfernen der gebundenen Hybridisierungssonden vom Träger und
(f5) gegebebenfalls Wiederholen der Schritte (f3) und (f4) mit weiteren nachweisbaren Hybridisierungssonden, die sich von den anderen Sonden dadurch unterscheiden, daß sie an andere Zielsequenzen binden.

17. Verfahren zur Umwandlung einer bereits vorhandenen Genbank in eine modifizierte Genbank, an der eine simultane Sequenzierung von Nukleinsäuren durchführbar ist,
**gekennzeichnet durch**
(a) Bereitstellen einer beliebigen Genbank, bestehend aus einer Vielzahl von unterschiedlichen DNA-Fragmenten, die in einen Basisvektor kloniert worden sind, wobei der Basisvektor in Nachbarschaft der einklonierten DNA-Fragmente mindestens eine singuläre, selten vorkommende enzymatische Spaltstelle enthält,
(b) Linearisieren der Genbank durch Spaltung an einer selten vorkommenden Spaltstelle gemäß (a),
(c) Aufteilen der Genbank in mehrere Gruppen,
(d) Ligieren der aufgeteilten, linearisierten Genbank mit einem doppelsträngigen DNA-Adaptor, wobei der doppelsträngige Bereich des Adaptors eine Länge von ≥ 5 Nukleotiden besitzt und an beiden Seiten Enden aufweist, die zur Spaltstelle des Basisvektors passend sind, wobei an jede Gruppe der Genbank ein Adaptor mit unterschiedlicher Sequenz ligiert wird, und
(e) gegebenenfalls Abtrennen der gewünschten Ligationsprodukte von Nebenprodukten.

18. Verfahren nach Anspruch 17,
**dadurch gekennzeichnet,** daß die Sequenz des Adaptors so gewählt wird, daß durch Einführung des Adaptors die Spaltstelle eliminiert wird.

19. Verfahren nach Anspruch 18,
**dadurch gekennzeichnet,** daß Schritt (e) ein Nachspalten der Ligationsprodukte aus (d) mit dem zum Linearisieren der Genbank verwendeten Enzym umfaßt.

20. Verfahren nach einem der Ansprüche 17 bis 19,
**dadurch gekennzeichnet,** daß man die Spaltung mit NotI, SfiI, RsrII, SgrAI, SwaI, PacI, AscI, PmeI, Sse8387I, SrsI oder I-SceI durchführt.

21. Verfahren nach einem der Ansprüche 17 bis 20,
**dadurch gekennzeichnet,** daß man ein Adaptormolekül verwendet, das eine symmetrische Sequenz aufweist.

22. Verfahren nach einem der Ansprüche 17 bis 20,
**dadurch gekennzeichnet,** daß man ein Adaptormolekül verwendet, das eine unsymmetrische Sequenz aufweist.

23. Verfahren nach Anspruch 17,
**dadurch gekennzeichnet,** daß man eine Genbank in einem Basisvektor verwendet, der auf beiden Seiten in Nachbarschaft der einklonierten DNA-Fragmente jeweils mindestens eine singuläre, selten vorkommende enzymatische Spaltstelle enthält, so daß die Schritte (b) bis (e) gemäß Anspruch 17 zweimal durchgeführt werden können.

## Claims

1. Method for the simultaneous sequencing of nucleic acids,
**wherein**
(a) numerous DNA fragments whose sequence is to be determined are prepared,
(b) the totality of the DNA fragments to be sequenced is divided into several groups,
(c) double-stranded DNA adaptors are ligated to both ends of the divided DNA fragments from (b) wherein the adaptors have a double-stranded region with a length of ≥ 5 nucleotides, have a first end that is compatible to the ends of the DNA fragments and a second end suitable for cloning into a vector and wherein adaptors having a different nucleotide sequence are ligated to each group of the divided DNA fragments,
(d) the DNA fragments from (c) which are provided at both ends with adaptors are cloned into a base vector which has restriction sites matching the second ends of the adaptors,
(e) a number of clones from (d) are selected, but however, only 1 clone at most may originate from one group and sequencing reactions are carried out on the selected clones and
(f) the results of the sequencing reactions are analysed using a number of detectable hybridization probes each of which are specific for only one single clone from (e).

2. Method as claimed in claim 1,
**wherein**
the DNA fragments have blunt ends.

3. Method as claimed in claim 1,
**wherein**
the DNA fragments have stepped ends.

4. Method as claimed in one of the claims 1 to 3,
**wherein**
adaptors are used whose second end is stepped.

5. Method as claimed in one of the claims 1 to 4,
**wherein**
the length of the double-stranded DNA adaptors is 10 to 40 nucleotides.

6. Method as claimed in one of the claims 1 to 5,
**wherein**
a single adaptor is used within each group.

7. Method as claimed in one of the claims 1 to 6,
**wherein**
two adaptors are used within each group.

8. Method as claimed in one of the claims 1 to 5 or 7,
**wherein**
for the ligation reaction within each group two adaptors with a different nucleotide sequence and stepped second ends are used and a base vector is used to clone the DNA fragments provided with adaptors which has two different restriction sites that are compatible to the respective second ends of the adaptors.

9. Method as claimed in claim 8,
**wherein**
the stepped second ends of the two adaptor molecules are not compatible with one another.

10. Method as claimed in one of the claims 1 to 9,
**wherein**
the base vector used for cloning the DNA fragments has in addition at least one rarely occurring enzymatic cleavage site on both sides of the insertion site.

11. Method as claimed in claim 10,
**wherein**
the rarely occurring enzymatic cleavage site has a recognition sequence of at least 7 nucleotides or/and contains the nucleotide sequence 5'-CG-3' at least once.

12. Method as claimed in claim 11,
**wherein**
the cleavage site is a NotI, SfiI, RsrII, SgrAI, SwaI, PacI, AscI, PmeI, Sse8387I, SrsI or I-SceI cleavage site.

13. Method as claimed in one of the claims 1 to 12,
**wherein**
a base vector is used to clone the DNA fragments provided with adaptors which has transcription terminators in the vicinity of the restriction sites used for the insertion of the DNA fragments.

14. Method as claimed in one of the claims 1 to 13,
**wherein**
non-radioactively labelled hybridization probes are used to analyse the sequencing reactions.

15. Method as claimed in claim 14,
**wherein**
biotin-, fluorescent-, luminescent-, digoxigenin- or/and enzyme-labelled hybridization probes are used.

16. Method as claimed in claim 1,
**wherein**
(f) comprises the following steps:
(f1) separating the sequencing products obtained by the sequencing reactions from (e) according to their size in which corresponding sequencing products of a particular reaction from several clones are separated together,
(f2) transferring the separated sequencing products onto a suitable carrier for binding nucleic acids and if desired immobilization of the transferred sequencing products on the carrier,
(f3) reversible hybridization of the carrier with (i) a first detectable hybridization probe or (ii) with two or several selectively detectable hybridization probes in which each probe is in each case only specific for a single clone from (e) and in which the specificity of each probe is defined by a hybridization carried out under the reaction conditions to a particular target sequence which is formed from a particular adaptor and if desired neighbouring vector sequences,
(f4) analysis of the results from (f3), removal of the bound hybridization probes from the carrier and
(f5) if desired, repetition of steps (f3) and (f4) with further detectable hybridization probes which differ from the other probes in that they bind to other target sequences.

17. Method for converting an already existing gene bank into a modified gene bank on which a simultaneous sequencing of nucleic acids can be carried out
**wherein**
(a) any desired gene bank is prepared consisting of a multitude of different DNA fragments which have been cloned into a base vector, in which the base vector contains at least one singular rarely occurring enzymatic cleavage site in the vicinity of the cloned DNA fragments,
(b) the gene bank is linearized by cleavage at a rarely occurring cleavage site according to (a),
(c) the gene bank is divided into several groups,
(d) the divided linearized gene bank is ligated with a double-stranded DNA adaptor, the double-stranded region of the adaptor having a length of ≥ 5 nucleotides and ends on both sides which match the cleavage site of the base vector, whereby an adaptor with a different sequence is ligated to each group of the gene bank and
(e) if desired, the desired ligation products are separated from by-products.

18. Method as claimed in claim 17,
**wherein**
the sequence of the adaptor is selected so that the cleavage site is eliminated by introduction of the adaptor.

19. Method as claimed in claim 18,
**wherein**
step (e) comprises recleaving the ligation products from (d) with the enzyme used to linearize the gene bank.

20. Method as claimed in one of the claims 17 to 19,
**wherein**
the cleavage is carried out with NotI, SfiI, RsrII, SgrAI, SwaI, PacI, AscI, PmeI, Sse8387I, SrsI or I-SceI.

21. Method as claimed in one of the claims 17 to 20,
**wherein**
an adaptor molecule is used which has a symmetrical sequence.

22. Method as claimed in one of the claims 17 to 20,
**wherein**
an adaptor molecule is used which has an unsymmetrical sequence.

23. Method as claimed in claim 17,
**wherein**
a gene bank in a base vector is used which contains at least one singular rarely occurring enzymatic cleavage site on both sides in the vicinity of the cloned DNA fragments so that steps (b) to (e) according to claim 17 can be carried out twice.

## Revendications

1. Procédé de clonage simultané d'acides nucléiques, caractérisé par
(a) la fourniture d'une multiplicité de fragments d'ADN dont la séquence doit être déterminée,
(b) la répartition de la totalité des fragments d'ADN à séquencer en plusieurs groupes,
(c) la ligature d'adaptateurs d'ADN double brin aux deux extrémités des fragments d'ADN répartis provenant de (b), où les adaptateurs possèdent un domaine double brin d'une longueur ≥ 5 nucléotides, présentent une première extrémité compatible avec les extrémités des fragments d'ADN et une seconde extrémité appropriée au clonage dans un vecteur, et où des adaptateurs de séquence nucléotidique différente sont ligaturés à chaque groupe des fragments d'ADN répartis,
(d) le clonage des fragments d'ADN munis d'adaptateurs aux deux extrémités provenant de (c) dans un vecteur de base qui présente des sites de restriction qui sont appropriés aux secondes extrémités des adaptateurs,
(e) le choix d'un certain nombre de clones à partir de (d), toutefois 1 seul clone au maximum pouvant provenir d'un groupe, et la mise en oeuvre de réactions de séquençage sur les clones choisis, et
(f) l'exploitation des résultats des réactions de séquençage au moyen d'un certain nombre de sondes d'hybridation pouvant être mises en évidence, qui ne sont spécifiques chacune que d'un seul clone provenant de (e).

2. Procédé selon la revendication 1 caractérisé en ce que les fragments d'ADN présentent des extrémités franches.

3. Procédé selon la revendication 1 caractérisé en ce que les fragments d'ADN présentent des extrémités cohésives.

4. Procédé selon l'une des revendications 1 à 3 caractérisé en ce que l'on utilise des adaptateurs dont la seconde extrémité est cohésive.

5. Procédé selon l'une des revendications 1 à 4 caractérisé en ce que la longueur des adaptateurs d'ADN double brin est de 10 à 40 nucléotides.

6. Procédé selon l'une des revendications 1 à 5 caractérisé en ce que l'on utilise un seul adaptateur à l'intérieur de chaque groupe.

7. Procédé selon l'une des revendications 1 à 6 caractérisé en ce que l'on utilise deux adaptateurs à l'intérieur de chaque groupe.

8. Procédé selon l'une des revendications 1 à 5 ou 7 caractérisé en ce que l'on utilise pour la réaction de ligature à l'intérieur de chaque groupe deux adaptateurs de séquence nucléotidique différente et ayant des secondes extrémités cohésives et en ce que l'on utilise pour le clonage des fragments d'ADN munis d'adaptateurs un vecteur de base qui présente deux sites de restriction différents, compatibles dans chaque cas avec les secondes extrémités des adaptateurs.

9. Procédé selon la revendication 8 caractérisé en ce que les secondes extrémités cohésives des deux molécules d'adaptateurs ne sont pas compatibles entre elles.

10. Procédé selon l'une des revendications 1 à 9 caractérisé en ce que le vecteur de base utilisé pour le clonage des fragments d'ADN présente en outre aux deux extrémités du site d'insertion au moins un site de clivage enzymatique peu fréquent.

11. Procédé selon la revendication 10 caractérisé en ce que le site de clivage enzymatique peu fréquent contient une séquence de reconnaissance d'au moins 7 nucléotides et/ou au moins une fois la séquence nucléotidique 5'-CG-3'.

12. Procédé selon la revendication 11 caractérisé en ce que le site de clivage est un site de coupure pour NotI, SfiI, RsrII, SgrAI, SwaI, PacI, AscI, PmeI, Sse8387I, SrsI ou I-SceI.

13. Procédé selon l'une des revendications 1 à 12 caractérisé en ce que l'on utilise pour le clonage des fragments d'ADN munis d'adaptateurs un vecteur de base qui présente des terminateurs de transcription au voisinage des sites de restriction utilisés pour l'insertion des fragments d'ADN.

14. Procédé selon l'une des revendications 1 à 13 caractérisé en ce que l'on utilise des sondes d'hybridation marquées de manière non radioactive pour l'exploitation des réactions de séquençage.

15. Procédé selon la revendication 14 caractérisé en ce que l'on utilise des sondes d'hybridation marquées avec la biotine, par fluorescence, par luminescence, avec la digoxigénine et/ou avec une enzyme.

16. Procédé selon la revendication 1 caractérisé en ce que (f) comprend les étapes suivantes:
(f1) séparation des produits de séquençage obtenus par les réactions de séquençage à partir de (e) selon leur taille, où des produits de séquençage correspondants d'une réaction déterminée sont séparés ensemble à partir de plusieurs clones,
(f2) transfert des produits de séquençage séparés sur un support approprié à la liaison d'acides nucléiques et éventuellement immobilisation sur le support des produits de séquençage transférés,
(f3) hybridation réversible du support avec (i) une première sonde d'hybridation pouvant être mise en évidence ou (ii) avec deux ou plusieurs sondes d'hybridation pouvant être mises en évidence sélectivement, où chaque sonde n'est spécifique dans chaque cas que d'un seul clone provenant de (e) et où la spécificité de chaque sonde est définie par une hybridation ayant lieu dans les conditions réactionnelles avec une séquence cible déterminée qui est formée à partir d'un adaptateur déterminé et éventuellement de séquences de vecteur voisines,
(f4) exploitation des résultats de (f3), retrait des sondes d'hybridation liées du support et
(f5) éventuellement répétition des étapes (f3) et (f4) avec des sondes d'hybridation pouvant être mises en évidence supplémentaires qui se distinguent des autres sondes par le fait qu'elles se lient à d'autres séquences cibles.

17. Procédé pour convertir une banque de gènes déjà existante en une banque de gènes modifiée sur laquelle un séquençage simultané d'acides nucléiques peut être réalisé, caractérisé par
(a) la fourniture d'une banque de gènes quelconque consistant en une multiplicité de fragments d'ADN différents qui ont été clonés dans un vecteur de base, où le vecteur de base contient au voisinage des fragments d'ADN clonés au moins un site de clivage enzymatique peu fréquent singulier,
(b) la linéarisation de la banque de gènes par clivage au niveau d'un site de clivage peu fréquent selon (a),
(c) la répartition de la banque de gènes en plusieurs groupes,
(d) la ligature de la banque de gènes linéarisée répartie avec un adaptateur d'ADN double brin, où le domaine double brin de l'adaptateur possède une longueur ≥ 5 nucléotides et présente des deux côtés des extrémités qui sont appropriées au site de clivage du vecteur de base, où un adaptateur de séquence différente est ligaturé à chaque groupe de la banque de gènes, et
(e) éventuellement la séparation des produits de ligature voulus d'avec des sous-produits.

18. Procédé selon la revendication 17 caractérisé en ce que la séquence de l'adaptateur est choisie de manière que le site de clivage soit éliminé par insertion de l'adaptateur.

19. Procédé selon la revendication 18 caractérisé en ce que l'étape (e) comprend un post-clivage des produits de ligature provenant de (d) avec l'enzyme utilisée pour la linéarisation de la banque de gènes.

20. Procédé selon l'une des revendications 17 à 19 caractérisé en ce que le clivage est réalisé avec NotI, SfiI, RsrII, SgrAI, SwaI, PacI, AscI, PmeI, Sse8387I, SrsI ou I-SceI.

21. Procédé selon l'une des revendications 17 à 20 caractérisé en ce que l'on utilise une molécule d'adaptateur qui présente une séquence symétrique.

22. Procédé selon l'une des revendications 17 à 20 caractérisé en ce que l'on utilise une molécule d'adaptateur qui présente une séquence dissymétrique.

23. Procédé selon la revendication 17 caractérisé en ce que l'on utilise une banque de gènes dans un vecteur de base qui contient des deux côtés au voisinage des fragments d'ADN clonés dans chaque cas au moins un site de clivage enzymatique peu fréquent singulier de sorte que les étapes (b) à (e) selon la revendication 17 peuvent être réalisées deux fois.
